# EUROPÄISCHE PATENTSCHRIFT

(11) **EP 3 853 199 B1**
(45) Veröffentlichungstag und Bekanntmachung des Hinweises auf die Patenterteilung: **25.10.2023**
(21) Anmeldenummer: 18778413.7
(22) Anmeldetag: 19.09.2018
(51) Int. Cl.: C07C 47/225, C11B 9/00, A61Q 13/00

(54) **2-(5-ISOPROPYL-2-METHYL-CYCLOHEX-2-EN-1-YL-) ACETALDEHYD UND 2-(6-ISOPROPYL-3-METHYL-CYCLOHEX-2-EN-1-YL-) ACETALDEHYD ALS NEUE RIECHSTOFFE**
2-(5-ISOPROPYL-2-METHYL-CYCLOHEX-2-EN-1-YL-) ACETALDEHYDE AND 2-(6-ISOPROPYL-3-METHYL-CYCLOHEX-2-EN-1-YL-) ACETALDEHYDE AS NEW ODORANTS
2-(5-ISOPROPYL-2-MÉTHYL-CYCLOHEX-2-ÉN-1-YL-)ACÉTALDÉHYDE ET 2-(6-ISOPROPYL-3-MÉTHYL-CYCLOHEX-2-ÉN-1-YL-)ACÉTALDÉHYDE EN TANT QUE NOUVEAUX PARFUMS

(43) Veröffentlichungstag der Anmeldung: 28.07.2021
(73) Patentinhaber: Symrise AG, 37603 Holzminden (DE)
(72) Erfinder: HÖLSCHER, Bernd, 37620 Halle (DE); GERSTMANN, Frank, 37603 Holzminden (DE); MANSFELD, Mark, 37647 Brevörde (DE)
(74) Vertreter: Global IP Europe Patentanwaltskanzlei
(86) Internationale Anmeldenummer: PCT/EP2018/075387
(87) Internationale Veröffentlichungsnummer: WO 2020/057741

(56) Entgegenhaltungen:
- EP-A1- 2 578 671
- EP-A2- 1 054 053
- EP-B1- 2 578 671
- EP-B1- 3 349 862
- WO-A1-2017/046071
- DE-A1- 10 114 176

## Beschreibung

### Gebiet der Erfindung

Die vorliegende Erfindung betrifft neue Verbindungen der Formel (**I**) 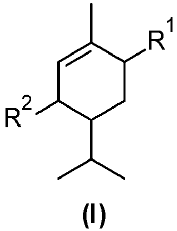 sowie Mischungen, umfassend oder bestehend aus Verbindungen der Formel (**I**). Darüber hinaus betrifft die vorliegende Erfindung ein Verfahren zur Herstellung der Verbindungen der Formel (**I**) und die Verwendung der Verbindungen der Formel (**I**) als Riechstoff, insbesondere als Maiglöckchen-Riechstoff. Letztendlich betrifft die vorliegende Erfindung Riechstoffzubereitungen und parfümierte Produkte bzw. Konsumgüter, die mindestens eine der Verbindungen der Formel (**I**) umfassen.

### Stand der Technik

Verbindungen mit blumigem Geruch sind eine unverzichtbare Komponente in der Riechstoffindustrie sowie bei der Herstellung von Parfüms, Kosmetika, Körperpflegemitteln sowie Wasch- und Reinigungsmitteln und sonstigen Konsumgütern. Trotz einer Vielzahl bereits vorhandener Riechstoffe besteht seitens der Parfümerie weiterhin eine große Nachfrage nach neuen blumigen Riechstoffen, insbesondere nach Maiglöckchen-Geruchsnoten, die eine Erweiterung der parfümistischen Palette ermöglichen. Maiglöckchen-Geruchsnoten zählen zu den beliebten Geruchsnoten und werden sehr oft in Parfüms und Duftstoffmischungen verwendet. Umfangreiche Übersichtsartikel über die historische Entwicklung von Maiglöckchen-Riechstoffen sind nachzulesen in den Publikationen A. Goeke, P. Kraft, D. Lelievre, Perfumer & Flavorist, 2018, 43(3), 24 und J. Coulomb, Perfumer & Flavorist, 2018, 43 (4), 40.

Aldehydische Cyclohexene sind in der Literatur als Riechstoffe beschrieben worden.

EP 1 054 053 A1 beschreibt tert-Butyl-substituierte Cyclohexene, die Maiglöckchen-artig riechen. Während Verbindung (**A**) aldehydisch, floral, nach Maiglöckchen und fettig riecht, zeichnet sich die Methyl-substituierte Verbindung (**B**) neben Maiglöckchen mit weißen blumigen Geruchsnoten aus.

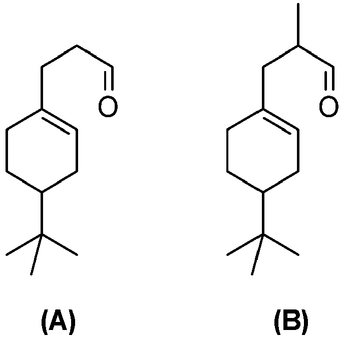

EP 2 578 671 A1 beschreibt Riechstoffe mit einer starken grünen, wässrigen und blumigen Geruchsnote, die an Maiglöckchen erinnern.

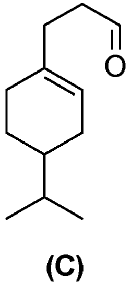

WO 2017046071 A1 beschreibt ebenfalls Cyclohexene mit grünen und blumigen Geruchsnoten.

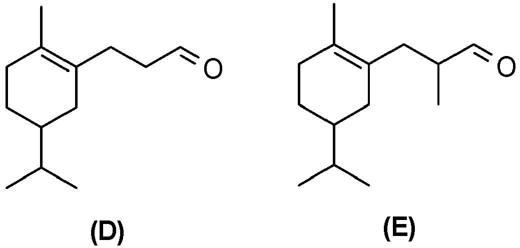

DE 101 14 176 A1 offenbart Cyclohexenacetaldehyde der allgemeinen Formel (F) 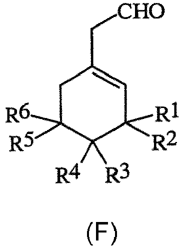 mit blumiger, Maiglöckchen-Note.

Allen beschriebenen Cyclohexenen (A) bis (E) ist gemein, dass sie einen Propanalsubstituenten aufweisen. Die Synthesen sind mehrstufig und verlaufen teilweise unter moderaten Ausbeuten. Darüber hinaus verläuft die Synthese dieser Verbindungen über Cyclohexen-Derivate mit exo-ständiger Doppelbindung, die selektiv herzustellen chemisch aufwendig sind.

An neue Riechstoffe werden sehr hohe Anforderungen gestellt: Neben ihren primären, nämlich geruchlichen Eigenschaften sollen sie zusätzliche positive Sekundäreigenschaften besitzen, wie z.B. eine höhere Stabilität unter bestimmten Anwendungsbedingungen, eine höhere Substantivität und/oder Diffusivität oder ein besseres Haftungsvermögen. Außerdem sollen sie eine sehr gute biologische Abbaubarkeit aufweisen und dermatologisch sowie toxikologisch unbedenklich sein.

Riechstoffe, die sich durch die oben erwähnten positiven Sekundäreigenschaften auszeichnen, ermöglichen eine erhöhte Effektivität bei der Herstellung von Riechstoffzubereitungen und parfümierten Produkten. So können z.B. durch den Einsatz von Riechstoffen mit einem besseren sensorischen Profil, einer höheren Substantivität und/oder einem besseren Haftungsvermögen die Anzahl und die Einsatzmengen von Riechstoffen in entsprechenden Formulierungen optimiert und/oder minimiert werden, was zu einer nachhaltigen Ressourcenschonung bei der Herstellung parfümierter Produkte führt.

Die primäre Aufgabe der vorliegenden Erfindung bestand darin, neue Verbindungen mit einer blumigen Geruchsnote, insbesondere einer Maiglöckchen-Geruchsnote, bereitzustellen, das heißt, die Verbindungen sollen einen Geruchseindruck vermitteln, der weitgehend der Komplexität des natürlichen Geruchs der Maiglöckchenblüte entspricht. Darüber hinaus sollten diese Verbindungen hinsichtlich ihrer Sekundäreigenschaften den aus dem Stand der Technik bekannten Maiglöckchen-Riechstoffen entsprechen oder überlegen sein. Außerdem sollten die neuen Riechstoffe zurückzuführen sein auf erneuerbare und natürliche Ausgangsmaterialien, d.h. nachwachsende Rohstoffe.

Eine weitere Aufgabe der vorliegenden Erfindung bezieht sich auf die Herstellung solcher neuartigen Verbindungen.

Eine zusätzliche Aufgabe betrifft die Bereitstellung von neuen Verbindungen mit einer blumigen Geruchsnote, insbesondere einer Maiglöckchen-Geruchsnote, zur Verwendung als Riechstoffe bzw. zur Herstellung von Riechstoffzubereitungen, sowie parfümierte Produkte bzw. Konsumgüter, die mindestens eine dieser neuen Verbindungen umfassen.

Die gestellten Aufgaben werden erfindungsgemäß durch die Gegenstände der unabhängigen Patentansprüche gelöst. Weitere Aspekte und bevorzugte Ausgestaltungen der vorliegenden Erfindung ergeben sich aus dem Wortlaut der abhängigen Patentansprüche, der nachfolgenden Beschreibung sowie den Ausführungsbeispielen.

### Detaillierte Beschreibung der Erfindung

Die Suche nach geeigneten Stoffen mit Maiglöckchen-Geruchsnote, die zur vorliegenden Erfindung führte, wurde durch folgende Sachverhalte erschwert:
- die Mechanismen der Geruchswahrnehmung sind nicht ausreichend bekannt; und
- die Zusammenhänge zwischen der speziellen Geruchswahrnehmung einerseits und der chemischen Struktur des zugehörigen Riechstoffs andererseits sind nicht hinreichend erforscht.

Weiterhin führen bereits geringfügige Änderungen am strukturellen Aufbau eines bekannten Riechstoffs zu starken Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Ein erster Gegenstand der vorliegenden Erfindung betrifft neue Verbindungen der allgemeinen Formel (I) 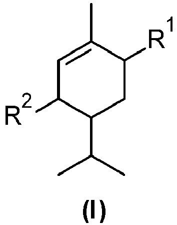 worin die variablen Reste R¹ und R² die folgende Bedeutung haben:
R¹ steht für H oder -CH₂-CHO, und
R² steht für H oder -CH₂-CHO,
mit der Maßgabe, dass R¹ ≠ R² ist;
sowie deren Stereoisomere oder Mischungen daraus.

Im Kontext der vorliegenden Erfindung werden unter der Bezeichnung "Verbindungen der Formel (I)" sowohl die einzelnen Verbindungen der Formel (I) als auch sämtliche Mischungen der Verbindungen der Formel (I) in jedem beliebigen Mischungsverhältnis verstanden. Das heißt, Ausführungen in der nachfolgenden Beschreibung betreffend "Verbindungen der Formel (**I**)" gelten sowohl für eine einzelne Verbindung der Formel (**I**) als auch für Mischungen, bestehend aus oder umfassend Verbindungen der Formel (**I**) in jedem Mischungsverhältnis.

Die Verbindungen der Formel (**I**) können erfindungsgemäß und bevorzugt in verschiedenen Formen vorliegen, entsprechend der möglichen Isomere, insbesondere der Regioisomere für R1 und R2 und/oder der Stereoisomere der Formel (**I**), und zwar einzeln sowie als Mischungen.

In einer bevorzugten Ausgestaltung der Erfindung ist ein Regioisomer eine Verbindung der Formel (**I**), worin R¹ eine -CH₂-CHO-Gruppe und R² ein Wasserstoffatom darstellt.

In einer weiteren bevorzugten Ausgestaltung der Erfindung ist ein weiteres Regioisomer eine Verbindung der Formel (**I**), worin R¹ ein Wasserstoffatom und R² eine -CH₂-CHO-Gruppe darstellt.

Die erfindungsgemäßen Verbindungen der Formel (**I**), wie sie oben definiert wurden, können auch als Stereoisomere vorliegen. Im Kontext der vorliegenden Erfindung umfasst der Begriff Stereoisomere alle möglichen Diastereomere oder Enantiomere der Verbindungen der Formel (**I**)**.**

Desweiteren schließt die Definition der Verbindungen der Formel (**I**) ebenso Mischungen der Stereoisomere ein, insbesondere auch die Racemate oder enantiomerenangereicherte Mischungen, sowie deren enantiomerenreine Formen.

Überraschenderweise hat sich gezeigt, dass die Verbindungen der Formel (**I**) einen Geruchseindruck vermitteln, der der Komplexität des natürlichen Geruchs der Maiglöckchenblüte sehr nahe kommt. Das heißt, der von den Verbindungen der Formel (**I**) vermittelte Geruchseindruck zeichnet sich durch eine herausragende Natürlichkeit und Komplexität aus, insbesondere hinsichtlich der Maiglöckchen-Geruchsnote.

Darüber hinaus betrifft die vorliegende Erfindung eine Verbindung der Formel (**I**), die ausgewählt ist aus der Gruppe, die besteht aus den Verbindungen der Formel (**II**) und Verbindungen der Formel (**III**): 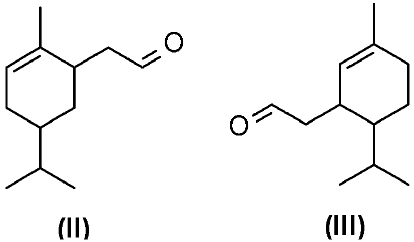 sowie deren Stereoisomere, insbesondere Diastereomere oder Enantiomere, oder Mischungen daraus.

Die erfindungsgemäßen Verbindungen der Formel (**II**) bzw. Formel (**III**) können als 2-(5-lsopropyl-2-methyl-cyclohex-2-en-1-yl-)acetaldehyd bzw. als 2-(6-Isopropyl-3-methyl-cyclohex-2-en-1-yl-)acetaldehyd bezeichnet werden.

Die erfindungsgemäßen Verbindungen der Formel (**II**) oder der Formel (**III**) können einzeln vorliegen sowie als Mischungen aus den vorgenannten Verbindungen der Formel (II) oder der Formel (III).

Die Verbindungen der Formel (**II**) zeichnen sich dadurch aus, dass sie insbesondere aldehydische, blumige, grüne, trockene und Maiglöckchen-artige Noten aufweisen. Die Verbindungen der Formel (**III**) weisen insbesondere grüne, aldehydische und blumige Geruchsnoten auf, die an Maiglöckchen erinnern.

Die Verbindungen der Formel (**II**) oder der Formel (**III**) können in verschiedenen Formen entsprechend der möglichen Stereoisomere, insbesondere Enantiomere oder Diastereomere, der Formel (**II**) oder der Formel (**III**) vorliegen.

Desweiteren schließt die Definition der Verbindungen der Formel (**II**) oder der Formel (**III**) ebenso Mischungen der Stereoisomere ein, insbesondere auch die Racemate oder enantiomerenangereicherte Mischungen, sowie deren enantiomerenreine Formen.

In einer weiteren bevorzugten Ausgestaltung gemäß dem ersten Aspekt betrifft die vorliegende Erfindung Verbindungen der Formel (**II**), wie sie durch die Formel (**IIa**) oder die Formel (**IIb**) wiedergegeben werden: 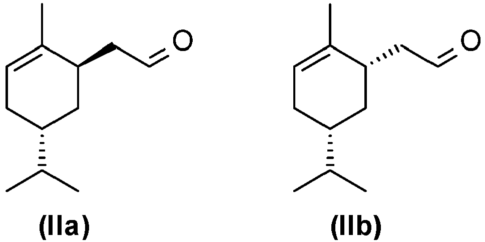 sowie deren Mischungen.

Die enantiomerenreine Verbindung der Formel (**IIa**) weist insbesondere Maiglöckchen-artige, blumige und grüne Noten auf. Die enantiomerenreine Verbindung der Formel (**IIb**) weist insbesondere blumige, grüne und aldehydische Noten auf.

In einer weiteren bevorzugten Variante betrifft die vorliegende Erfindung Verbindungen der Formel (**III**)**,** wie sie durch die Formel (**IIIa**) oder die Formel (**IIIb**) wiedergegeben werden: 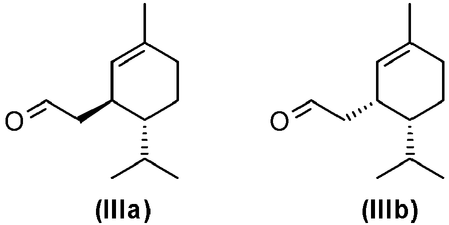 sowie deren Mischungen.

Die enantiomerenreine Verbindung der Formel (**IIIa**) weist insbesondere aldehydische, grüne und blumige Noten auf, die an Maiglöckchen erinnern.

Die enantiomerenreine Verbindung der Formel (**IIIb**) weist insbesondere grüne, blumige, aldehydische und Maiglöckchen-artige Noten auf.

Vorzugsweise liegen die erfindungsgemäßen und zuvor beschriebenen Verbindungen der Formel (**IIa**), der Formel (**IIb**), der Formel (**IIIa**) oder der Formel (**IIIb**) als Racemate, enantiomerenangereicherte Mischungen oder in ihrer enantiomerenreinen Form vor.

Im vorliegenden Text werden von der Bezeichnung "Verbindungen der Formel (**IIa**)" bzw. "Verbindungen der Formel (**IIb**)" bzw. "Verbindungen der Formel (**IIIa**)" bzw. "Verbindungen der Formel (**IIIb**)" sowohl die einzelnen Verbindungen als auch sämtliche Mischungen aus den zuvorgenannten Verbindungen in jedem beliebigen Mischungsverhältnis umfasst.

Von der Bezeichnung "Verbindungen der Formel (**I**)" werden sowohl die einzelnen Verbindungen, ausgewählt aus der Gruppe, bestehend aus den Verbindungen der Formel (**II**), Verbindungen der Formel (**III**), Verbindungen der Formel (**IIa**) bzw. Formel (**IIb**) und Verbindungen der Formel (**IIIa**) bzw. Formel (**IIIb**), als auch deren Stereoisomere, sowie sämtliche Mischungen aus den zuvorgenannten Verbindungen und deren Stereoisomere in jedem beliebigen Mischungsverhältnis umfasst.

Überraschend weisen die erfindungsgemäßen Verbindungen vorwiegend Maiglöckchen-artige, blumige und grüne Noten auf.

Die Erfindung betrifft die erfindungsgemäßen Verbindungen als solche einzeln oder auch Mischungen der erfindungsgemäßen Verbindungen.

Ein weiterer Gegenstand der vorliegenden Erfindung betrifft somit Mischungen, umfassend Verbindungen der Formel (**I**), insbesondere (a) mindestens eine Verbindung der Formel (**II**) oder ein Stereoisomer davon, wie oben definiert, und (b) mindestens eine Verbindung der Formel (**III**) oder ein Stereoisomer davon, wie oben definiert, oder bestehend aus Verbindungen der Formel (**I**), insbesondere (a) mindestens einer Verbindung der Formel (**II**) oder ein Stereoisomer davon, wie oben definiert, und (b) mindestens einer Verbindung der Formel (**III**) oder ein Stereoisomer davon, wie oben definiert.

Unter den Mischungen, die die Verbindungen der Formel (**II**), wie oben definiert, und (ii) die Verbindung der Formel (**III**), wie oben definiert, umfassen oder aus diesen beiden Verbindungen bestehen, sind erfindungsgemäß Mischungen bevorzugt mit
- einem Massenverhältnis der Verbindung der Formel (**II**) zu der Verbindung der Formel (**III**) im Bereich von 0,01 bis 99,99, vorzugsweise im Bereich von 0,1 bis 99,9, bevorzugt im Bereich von 0,5 bis 99,5, bevorzugt im Bereich von 1,0 bis 99,0, bevorzugt im Bereich von 1,5 bis 98,5, bevorzugt im Bereich von 2,0 bis 98,0;
   und/oder
- einem Gesamtanteil der Verbindungen der Formeln (**II**) und (**III**) größer als 0,01 Gew.-%, vorzugsweise größer als 1 Gew.-%, bevorzugt größer als 10 Gew.-%, weiter bevorzugt größer als 50 Gew.-%, besonders bevorzugt größer als 90 Gew.-%, bezogen auf das Gesamtgewicht der Mischung, umfassend Verbindung (**II**) und/oder (**III**).

In einem weiteren Aspekt der vorliegenden Erfindung umfasst diese auch Herstellungsverfahren zur Herstellung der Verbindungen der Formel (**I**), Verbindungen der Formel (**II**) sowie Verbindungen der Formel (**III**), wobei mehrere alternative Syntheserouten möglich sind.

Die erfindungsgemäßen Verbindungen der Formel (**I**) bzw. der Formel (**II**) lassen sich durch die nachfolgend näher beschriebenen Verfahren synthetisieren.

Eine Möglichkeit zur Herstellung der erfindungsgemäßen Verbindungen der Formel (**I**) bzw. der Formel (**II**) besteht in der sauren Umsetzung der bekannten Verbindung 2-Methyl-5-(1-methylethyl-)2-cyclohexen-1-ol (**IV**) mit Vinylethern (R-O). Die daraus erhaltenen Acetale (**V**) werden sauer katalysiert in die Vinylether (**VI**) gespalten, die direkt thermisch in die gewünschten Aldehyde überführt werden. Die Reste R des Vinylethers sind ausgewählt aus der Gruppe, die besteht aus linearem C1- bis C20-Alkylrest, verzweigtem C1- bis C20-Alkylrest, Cyclopentylrest und Cyclohexylrest.

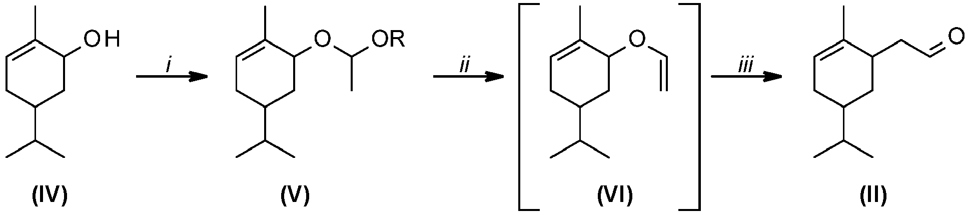

Eine weitere alternative Möglichkeit besteht in der sauren Umsetzung des bekannten und kommerziell erhältlichen Naturstoffes 2-Methyl-5-(1-methylethenyl-)2-cyclohexen-1-ol (**VII**) (E. Klein, G. Ohloff, Tetrahedron, 1963, 19, 1091; A. F. Thomas, G. Ohloff, Hel. Chim. Acta 1970, 53 (5), 1145) mit Vinylethern (R-O). Die daraus erhaltenen Acetale (**VIII**) werden sauer katalysiert in die Vinylether (**IX**) gespalten, die direkt thermisch in den Aldehyd (**X**) überführt werden. Eine Hydrierung der terminalen Doppelbindung ermöglicht die Darstellung des gewünschten Aldehyds (**II**). Die Reste R des Vinylethers sind ausgewählt aus der Gruppe, die besteht aus linearem C1- bis C20-Alkylrest, verzweigtem C1- bis C20-Alkylrest, Cyclopentylrest und Cyclohexylrest.

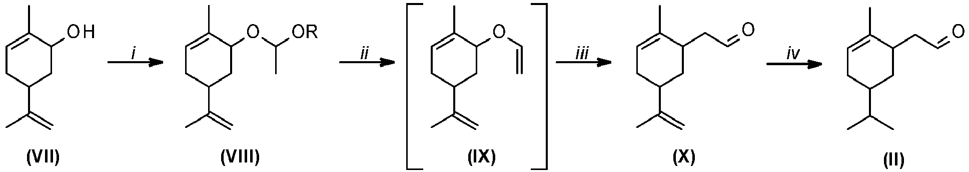

Die erfindungsgemäßen Verbindungen der Formel (**I**) bzw. der Formel (**III**) lassen sich durch die nachfolgend näher beschriebenen Verfahren synthetisieren.

Eine Möglichkeit zur Herstellung der erfindungsgemäßen Verbindungen der Formel (**I**) bzw. der Formel (**III**) besteht in der sauren Umsetzung des bekannten Naturstoffes 1-Methyl-4-(1-methylethyl-)2-cyclohexen-1-ol (**XI**) (E. Klein, G. Ohloff, Tetrahedron, 1963, 19, 1091; A. F. Thomas, G. Ohloff, Hel. Chim. Acta, 1970, 53 (5), 1145.) mit Vinylethern (R-O). Die daraus erhaltenen Acetale (**XII**) werden sauer katalysiert in die Vinylether (**XIII**) gespalten, die direkt thermisch in die gewünschten Aldehyde überführt werden. Die Reste R des Vinylethers sind ausgewählt aus der Gruppe, die besteht aus linearem C1- bis C20-Alkylrest, verzweigtem C1- bis C20-Alkylrest, Cyclopentylrest und Cyclohexylrest.

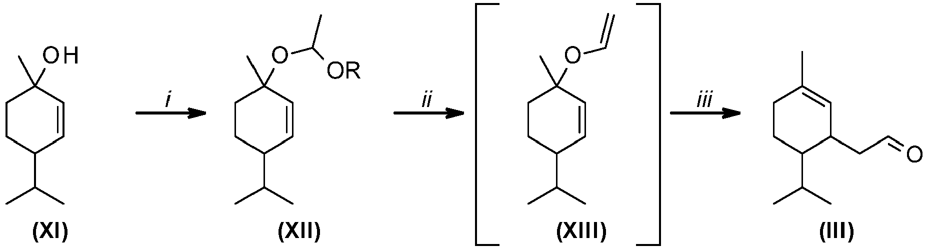

Eine weitere alternative Möglichkeit besteht in der sauren Umsetzung der bekannten Verbindung 1-Methyl-4-(1-methylethenyl-)2-cyclohexen-1-ol (**XX**) mit Vinylethern. Die daraus erhaltenen Acetale (**XXI**) werden sauer katalysiert in die Vinylether (**XXII**) gespalten, die direkt thermisch in den Aldehyd (**XXIII**) überführt werden. Eine Hydrierung der terminalen Doppelbindung ermöglicht die Darstellung des gewünschten Aldehyds (**III**). Die Reste R des Vinylethers sind ausgewählt aus der Gruppe, die besteht aus linearem C1- bis C20-Alkylrest, verzweigtem C1- bis C20-Alkylrest, Cyclopentylrest und Cyclohexylrest.

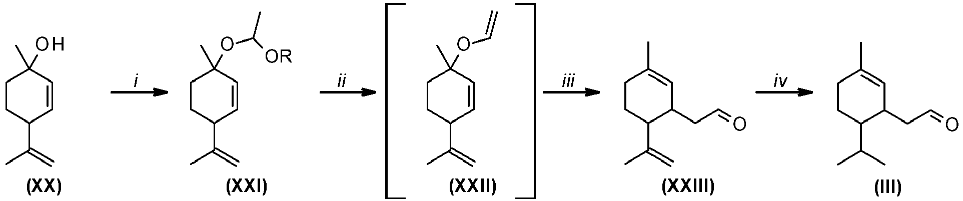

In Schritt (i) der oben beschriebenen erfindungsgemäßen Verfahren werden die Verbindungen mit den Formeln (**IV**), (**VII**), (**XI**) und (**XX**) in die Acetale überführt. Hierzu werden vorzugsweise Brranstedtsäuren oder eine Lewissäuren als Katalysator eingesetzt.

In Schritt (i) der oben beschriebenen erfindungsgemäßen Verfahren sind die Reste R der Verbindungen der Formeln (**V**), (**VIII**), (**XII**) und (**XXI**) definiert als vorzugsweise lineares Alkyl mit einer Kettenlänge von C1 bis C20, verzweigtes Alkyl mit einer Kettenlänge von C1 bis C20, Cyclopentyl oder Cyclohexyl.

Die Schritte (ii) und (iii) der oben beschriebenen erfindungsgemäßen Verfahren werden vorzugsweise als Eintopfverfahren durchgeführt. Dabei wird sauer zu den Vinylethern (**VI**), (**IX**), (**XIII**) und (**XXII**) gespalten und anschließend bei einer Temperatur ≥ 150 °C, vorzugsweise bei einer Temperatur im Bereich von 150 bis 250 °C, noch mehr bevorzugt bei einer Temperatur im Bereich von 160 bis 180 °C, die Claisen-Umlagerung zu den Verbindungen (**II**), (**III**), (**X**) und (**XXIII**) vollzogen. Als Katalysator werden vorzugsweise Brranstedtsäuren oder Lewissäuren eingesetzt.

Die Schritte (iv) der oben beschriebenen erfindungsgemäßen Verfahren werden in Gegenwart von Wasserstoff und eines Katalysators, beispielsweise von Palladium aufgebraucht auf ein Trägermaterial, unter den üblichen Bedingungen durchgeführt. Als vorteilhafte Trägermaterialien seien genannt: Aktivkohle, Kohle, Aluminiumoxide, Metalloxide, Kieselgele, Zeolithe,Tone, Tongranulate, amorphe Aluminiumsilicate, oder sonstige anorganische Träger. Ein bevorzugtes Trägermaterial ist Aktivkohle. Ein ganz besonders bevorzugter Katalysator ist Palladium auf Aktivkohle.

Des Weiteren betrifft die vorliegende Erfindung die Verwendung mindestens einer der erfindungsgemäßen Verbindungen, insbesondere der oben beschriebenen Verbindungen der Formeln (**I**), (**II**) oder (**III**) oder ein Stereoisomer davon, oder einer Mischung aus den vorgenannten Verbindungen oder Stereoisomeren davon als Riechstoff, insbesondere als Maiglöckchenduft, oder zur Herstellung einer Riechstoffzubereitung, insbesondere mit Maiglöckchenduft.

Ein weiterer Gegenstand der vorliegenden Erfindung betrifft somit eine Riechstoffzubereitung, die wenigstens eine Verbindung der Formeln (I), (II) oder (III) oder ein Stereoisomer davon, wie oben definiert, umfasst, vorzugsweise in einer sensorisch wirksamen Menge.

Eine Riechstoffzubereitung ist im Rahmen der vorliegenden Erfindung eine Mischung verschiedener Stoffe, welche gemäß einem Rezept oder einer Rezeptur nach einem vorgegebenen Verfahren aus den entsprechenden Stoffen erzeugt wird. Solche Zubereitungen werden gezielt zu dem Zweck hergestellt und eingesetzt, einen gewünschten, üblicherweise als angenehm oder in einer sonstigen Weise positiv empfundenen Geruchseindruck zu vermitteln, zu modifizieren oder zu verstärken.

Eine erfindungsgemäße Riechstoffzubereitung, insbesondere in Form eines Parfümöls, vorzugsweise mit einer Maiglöckchen-Geruchsnote, besteht aus mindestens einer oder umfasst mindestens eine erfindungsgemäße(n) Verbindung, insbesondere eine Verbindung der Formeln (I), (II) oder (III) oder ein Stereoisomer davon, wie oben definiert, und einen, zwei, drei, vier, fünf, sechs, sieben, acht, neun, zehn oder mehr weitere Riechstoff(e) und/oder Aromastoff(e), der/die keine Verbindung(en) der Formeln (**I**), (**II**) oder (**III**) sind.

In einer bevorzugten Ausführungsform umfasst oder besteht die erfindungsgemäße Riechstoffzubereitung eine Verbindung der Formel (**II**) und eine Verbindung der Formel (**III**) oder ein Isomer dieser Verbindungen.

Durch Kombination der Verbindung(en) der Formeln (**I**), (**II**) oder (**III**), wie oben definiert, vorzugsweise in einer der als bevorzugt angegebenen Ausgestaltungen, mit einem oder mehreren weiteren Riechstoff(en) und/oder Aromastoff(en) lassen sich interessante neue Riechstoffzubereitungen herstellen. Auf diese Weise lassen sich Mischungen mit besonders interessanten, natürlichen, neuen und originellen Noten kreieren. Riechstoffe und/oder Aromastoffe, die im Sinne der obigen Definition als weitere Riechstoffe oder Aromastoffe für den Einsatz in einer erfindungsgemäßem Riechstoffzubereitung geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercapto hexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal; 7-Methoxy-3,7-dimethyloctanal; 2,6,10-Trimethyl-9-undecenal; Geranylaceton; sowie die Dimethyl- und Diethylacetale von Geranial, Neral;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-lonon; beta-lonon; alphan-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl-)2-buten-1-on; 1,3,4,6,7,8a-Hexahydro-1,1,5,5-tetramethyl-2H-2,4a-methano-naphthalen-8-(5H)-on; 2-Methyl-4-(2,6,6-trimethyl-1-cyclohexen-1-yl-)2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl-)ethanol; 2-Methyl-4-(2,2,3-trimethyl-3-cyclopent-1-yl-)butanol; 2-Methyl-4-(2,2,3-trimethyl-3-cyclopent-1-yl-)2-buten-1-ol; 3-Methyl-5-(2,2,3-trimethyl-3-cyclopent-1-yl-)pentan-2-ol; 3-Methyl-5-(2,2,3-trimethyl-3-cyclopent-1-yl-)4-penten-2-ol; 3,3-Dimethyl-5-(2,2,3-trimethyl-3-cyclopent-1-yl-)4-penten-2-ol; 1-(2,2,6-Trimethylcyclohexyl-)pentan-3-ol; 1-(2,2,6-Trimethylcyclohexyl-)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy-)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1b]furan; 1,5,9-Trimethyl-13-oxabicyclo[10.1.0]trideca-4,8-dien; Rosenoxid; 2-(2,4-Dimethyl-3-cyclohexen-1-yl-)5-methyl-5-(1-methylpropyl-)1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl-)3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2-Methyl-4-(2,2,6-trimethyl-cyclohexen-1-yl-)2-butenal; 4-(4-Hydroxy-4-methylpentyl-)3-cyclohexencarbaldehyd; 4-(4-Methyl-3-penten-1-yl-)3-cyclohexencarbaldehyd;
der cycloaliphatischen Ketone wie z. B. 1-(3,3-Dimethylcyclohexyl-)4-penten-1-on; 2,2-Dimethyl-1-(2,4-dimethyl-3-cyclohexen-1-yl-)1-propanon; 1-(5,5-Dimethyl-1-cyclohexen-1-yl-)4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl-)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5- bzw. 6-inden-ylisobutyrat; 4,7-Methanooctahydro-5- bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der aliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-lsopropylphenyl)ethanol;
der Ester von aliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-T richlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der aliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;
der aromatischen und aliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl-)2,2-dimethylpropanal; 2-Methyl-3-(4-isopropylphenyl-)propanal; 2-Methyl-3-(4-isobutylphenyl-)propanal; 3-(4-tert.-Butylphenyl-)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl-)propanal; 2-Methyl-3-(4-methylendioxyphenyl-)propanal;
der aromatischen und aliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl-)2-butanon; 1-(2-Naphthalenyl-)ethanon;2-Benzofuranylethanon;(3-Methyl-2-benzofuranyl-)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl-)1H-5-indenyl]ethanon; 5`,6`,7`,8`-Tetrahydro-3`,5`,5`,6`,8`,8`-hexamethyl-2-aceto-naphthon;
der aromatischen und aliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl-)propanal oder 2,4-Dimethyl-3-cyclohexencarbaldehyd; 6-lsopropyl-chinolin; 6-Isobutylchinolin; 6-sec.-Butylchinolin;2-(3-Phenylpropyl-)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; 2,3-Dihydrocumarin; Octahydrocumarin.

In einer bevorzugten Ausführungsform umfasst eine erfindungsgemäße Riechstoffzubereitung, insbesondere in Form eines Parfümöls, einen, zwei oder mehr erfindungsgemäße Riechstoff(e) der Formeln (I), (II) oder (III) mit einer Maiglöckchen-Geruchsnote und optional einen oder mehrere weitere Riechstoffe mit einer Geruchsnote ausgewählt aus der Gruppe, die besteht aus blumig, süss, wässrig, pudrig.

In einer erfindungsgemäßen Riechstoffzubereitung, insbesondere eines Parfümöls, liegt die Menge der wenigstens einen Verbindung der Formeln (I), (II) oder (III) oder ihres Isomers wie oben definiert, im Bereich von 0,0001 bis 40 Gew.-%, vorzugsweise im Bereich von 0,001 bis 25 Gew.-%, bezogen auf das Gesamtgewicht der Riechstoffzubereitung. Neben dem Einsatz als Flüssigkeit in Lösungen oder in Emulsionen können die erfindungsgemäßen Verbindungen oder diese enthaltende Riechstoffzubereitungen, beispielsweise Parfümöle, an Feststoffen adsorbiert oder in Trägerstoffen (mikro)verkapselt eingesetzt werden. Diese Darreichungsformen können sowohl für eine feine Verteilung der Riechstoffe im Produkt als auch für eine kontrollierte Freisetzung bei der Anwendung sorgen. Derartige Feststoffe können poröse anorganische Materialien wie Leichtsulfat, Kieselgele, Zeolithe, Gipse, Tone, Tongranulate, Gasbeton usw. oder organische Materialien wie Hölzer, Cellulosebasierende Stoffe, Zucker oder Kunststoffe wie PVC, Polyvinylacetate oder Polyurethane sein.

Verkapselungsprodukte können beispielsweise sprühgetrocknet, als Einschluss-Komplex oder als Extrusions-Produkt vorliegen. Die Mikroverkapselung der Parfümöle kann beispielsweise durch das sogenannte Koazervationsverfahren mit Hilfe von Kapselmaterialien z.B. aus polyurethanartigen Stoffen oder Weichgelatine erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von dem Parfümöl und Cyclodextrinen oder Harnstoffderivaten in ein geeignetes Lösungsmittel, z.B. Wasser, hergestellt werden.

Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Die Adsorbate als auch die Verkapselungsprodukte können durch sogenanntes Coaten mit geeigneten Materialien im Hinblick auf eine gezieltere Duftfreisetzung weiter optimiert werden, wozu vorzugsweise wachsartige Kunststoffe wie z.B. Polyvinylalkohol verwendet werden können.

Gemäß einer bevorzugten Ausführungsform der vorliegenden Erfindung liegt in Parfümöl-Kompositionen, die an einem Trägerstoff adsorbiert eingesetzt werden, die eingesetzte Menge der erfindungsgemäßen Verbindungen der Formeln (I), (II) oder (III) oder ihrer Isomere wie oben definiert, im Bereich von 0,00001 bis 5 Gew.-%, vorzugsweise im Bereich von 0,00001 bis 1 Gew.-%, und besonders bevorzugt im Bereich von 0,0005 bis 0,01 Gew.-%, bezogen auf das Gesamtgewicht der Parfümöl-Komposition.

Die als Riechstoff zu verwendenden erfindungsgemäßen Verbindungen, wie sie oben definiert wurden, sowie die Mischungen daraus, sowie die erfindungsgemäßen Riechstoffzubereitungen, wie sie oben definiert wurden, werden insbesondere zur Herstellung parfümierter Produkte eingesetzt.

Dementsprechend betrifft die vorliegende Erfindung auch parfümierte Produkte bzw. Konsumgüter, einschließlich deren Vor- und Zwischenprodukte, welche einen erfindungsgemäßen Riechstoff oder eine erfindungsgemäße Riechstoffzubereitung umfassen. Ein erfindungsgemäßes parfümiertes Produkt bzw. Konsumgut umfasst die erfindungsgemäßen Verbindungen, wie sie oben definiert wurden, vorzugsweise in einer sensorisch wirksamen Mengen. Dabei kommt die erfindungsgemäße Verbindung entweder einzeln zum Einsatz oder ist Bestandteil einer erfindungsgemäßen Mischung bzw. Riechstoffzubereitung.

Parfümierte Produkte bzw. Konsumgüter enthalten oft eine Vielzahl unterschiedlicher chemischer Verbindungen. Der Einsatz der erfindungsgemäßen Verbindungen ist insbesondere vorteilhaft wegen deren geringer Reaktivität, so dass weder die erfindungsgemäßen Verbindungen noch die Inhaltsstoffe der Konsumgüter nachteilig verändert werden. Zudem beeinflussen die erfindungsgemäßen Verbindungen in den zur Erzielung der erwünschten Duftwirkung notwendigen Konzentrationen nicht nachteilig die physikalischen oder physikochemischen Eigenschaften der Konsumgüter (z.B. die Viskosität oder den pH-Wert).

Bei den in parfümierten Produkten bzw. Konsumgütern enthaltenen Stoffen handelt es sich oftmals um Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate. Mit diesen Stoffen weisen die erfindungsgemäßen Verbindungen in Mischungen eine hohe Kompatibilität auf.

Der Mengenanteil des erfindungsgemäßen Riechstoffs, d.h. der erfindungsgemäßen Verbindungen der Formeln (**I**), (**II**) oder (**III**) oder ihrer Isomere wie oben definiert, oder der erfindungsgemäßen Riechstoffzubereitung in dem parfümierten Produkt bzw. den Konsumgütern beträgt 0,0001 bis 5 Gew.-%, bevorzugt 0,001 bis 2 Gew.-% und besonders bevorzugt 0,001 bis 1 %.

Parfümierte Produkte bzw. Konsumgüter im Sinne der Erfindung sind Wasch- und Reinigungsmitteln, Hygiene- oder Pflegeprodukten, insbesondere aus dem Bereich der Körper- und Haarpflege, der Kosmetik, des Haushalts, Fine Fragrance (Parfüm), Air Care und Lufterfrischer, insbesondere Weichspüler, Bleichmittel, Desinfektionsmittel, Duftfreigabesysteme, Sonnenschutzmittel für die Haut, Haarpflegemittel und Deodorants. Besonders handelt es sich bei Konsumgütern um saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendesinfektionsmittel sowie Luftverbesserer in flüssiger, gelartiger oder auf einem festen Träger aufgebrachter Form einschließlich zur Beduftung von Toiletten, Aerosolsprays, Wachse und Polituren wie Möbelpolituren, Fußbodenwachse, Schuhcremes sowie Körperpflegemittel wie z.B. feste und flüssige Seifen, Duschgele, Shampoos, Rasierseifen, Rasierschäume, Badeöle, kosmetische Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und - lotionen, After-Shaves, After Shave-Cremes und Lotionen, Pre-Shaves, Bräunungscremes und -lotionen, Haarpflegeprodukte wie z.B. Haarsprays, Haargele, Haarlotionen, Haarspülungen, permanente und semipermanente Haarfärbemittel, Haarverformungsmittel wie Kaltwellen und Haarglättungsmittel, Haarwässer, Haarcremes und -lotionen, Erfrischungstücher, Deodorantien und Antiperspirantien wie z.B. Achselsprays, Roll-ons, Deosticks, Deocremes oder Produkte der dekorativen Kosmetik wie zum Beispiel Make-Up.

Letztendlich betrifft die vorliegende Erfindung betrifft auch ein Verfahren zur Parfümierung von parfümierten Produkten bzw. Konsumgütern, umfassend das Hinzufügen mindestens einer der erfindungsgemäßen Verbindung oder der erfindungsgemäßen Riechstoffzubereitungen von zu parfümierenden Produkten bzw. Konsumgütern. Der Vorteil der erfindungsgemäßen Verbindungen oder der erfindungsgemäßen Riechstoffzubereitungen bei diesem Verfahren ist, dass sie sich leicht in Konsumgüter einarbeiten lassen.

Die Verbindungen der vorliegenden Erfindung, wie sie oben beschrieben wurden, zeichnen sich dadurch aus, dass sie einen Geruchseindruck vermitteln, der weitgehend der Komplexität des natürlichen Geruchs der Maiglöckchenblüte entspricht. Darüber hinaus lassen sie sich aus erneuerbaren und natürlichen Ausgangsmaterialien, d.h. nachwachsenden Rohstoffen herstellen. Ein weiterer Vorteil der erfindungsgemäßen Verbindungen ist ihre leichte Zugänglichkeit: ihre Synthese erfolgt auf der Basis von endo-cyclischen Cyclohexenen anstelle von kompliziert hergestellten exo-Cyclohexenen.

### Beispiele

Die vorliegende Erfindung wird nachstehend anhand von Ausführungsbeispielen näher beschrieben.

### Ausführungsbeispiel 1: Herstellung von 2-(5-Isopropyl-2-methyl-cyclohex-2-en-1-yl)acetaldehyd

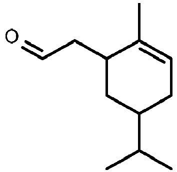

In einem 1 Liter-Doppelwandgefäß mit Kopfrührer, Thermostatanschluss, Kühler und Stickstoffatmosphäre werden 100 g 5-lsopropyl-2-methyl-cyclohex-2-en-1-ol (0,65 mol) und 0,4 g 85-%ige Phosphorsäure vorgelegt. Dann werden 98 g Butylvinylether (0,98 mol), in 1 bis 2 Stunden bei 35 °C zugetropft. Zur Nachreaktion wird noch 6 Stunden bei 35 °C nachgerührt. Der Ansatz wird bei Raumtemperatur mit 5-%iger Sodalösung und dann mit 5-%iger Natriumchloridlösung gewaschen und anschließend eingedampft. Die Rohausbeute beträgt 165 g.

Das Rohprodukt wird in einem 500 ml-Dreihalskolben mit Magnetrührer, 20 cm-Vigreuxkolonne und Destillationsbrücke, zusammen mit 165 g Dibenzylether und 6,5 g Capronsäure vorgelegt und bei 90 mbar aufgeheizt. Bei 160 - 175 °C werden die entstehenden Leichtsieder innerhalb von 5 Stunden bis zu einer Kopftemperatur von ca. 80 °C abdestilliert. Anschließend erfolgt die Destillation des Rohprodukts (Sumpftemperatur: 125 - 170 °C, Kopftemperatur: 70 - 110 °C, Vakuum: 1 mbar). Es werden 72 g abdestilliert. Nach DB-1-GC bzw. GCMS sind 60 % Produkt als cis/trans-Isomere entstanden. Die Trennung der beiden Isomeren erfolgt an einer Spaltrohrkolonne (Fischer Spaltrohrkolonne ^{®} HMS 500 AC). Siedepunkte bei 1 mbar:
2-(5-lsopropyl-2-methyl-cyclohex-2-en-1-yl)acetaldehyd: 71 °C, 2-(5-Isopropyl-2-methyl-cyclohex-2-en-1-yl)acetaldehyd: 73 °C.
2-(5-lsopropyl-2-methyl-cyclohex-2-en-1-yl)acetaldehyd: m/z : 43 (11), 55 (14), 67 (16), 81 (28), 93 (100), 109 (6), 119 (10), 136 (45), 147 (1), 180 (5) Geruchsbeschreibung: Aldehydisch, blumig nach Maiglöckchen, grün.
2-(5-Isopropyl-2-methyl-cyclohex-2-en-1-yl)acetaldehyd: m/z: 43 (13), 55 (17), 67 (21), 81 (33), 93 (100), 109 (9), 119 (13), 136 (55), 147 (2), 180 (4) Geruchsbeschreibung: grün, blumig nach Maiglöckchen, aldehydisch.

### Ausführungsbeispiel 2: Herstellung von 2-(6-Isopropyl-3-methyl-cyclohex-2-en-1-yl)acetaldehyd

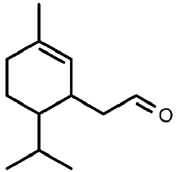

In einem 2 Liter-Rührwerk werden 122 g (0,8 mol) Piperiton und 700 ml Methanol vorgelegt. Bei 0 - 5 °C werden 80 g 25-%ige (0,5 mol) Natronlauge langsam zugegeben. Die Temperatur steigt auf ca. 20 °C an. Unter leichter Kühlung werden 181 g 30-%iges (1,6 mol) Wasserstoffperoxid in 1 h, bei 28 - 33 °C zugetropft. Anschließend wird noch 6 h bei 28 °C nachgerührt. Dann wird auf 1000 ml Wasser gegossen und zweimal mit je 300 ml MTBE extrahiert. Die MTBE-Phasen werden vereinigt und nacheinander mit Wasser, 10-%iger Kaliumjodidlösung und Wasser peroxidfrei gewaschen und dann eingedampft. Es werden 130 g Rohprodukt erhalten. Nach GC/GCMS sind trans-Piperitonepoxid und cis-Piperitonepoxid in einem Verhältnis von 1 : 3,5 enthalten.

Das Rohprodukt wird in einem 2 Liter-Rührwerk zusammen mit 500 ml Methanol vorgelegt und bei 0 - 5 °C mit 50 g (1 mol) Hydrazinmonohydrat versetzt. Die Temperatur steigt auf 17 - 20 °C an. Unter Kühlung und Stickstoffentwicklung werden 60 g (1 mol) Essigsäure in 30 min bei 20 - 25 °C zugetropft. Es wird noch für 2 h nachgerührt. Der Ansatz wird auf 1500 ml Wasser gegossen und dann zweimal mit je 500 ml MTBE extrahiert. Die MTBE-Phasen werden vereinigt und nacheinander mit Wasser und gesättigter Natriumhydrogencarbonat gewaschen und dann eingedampft. Es werden 99 g Rohprodukt erhalten. Nach GC/GCMS sind trans-4-lsopropyl-1-methyl-cyclohex-2-en-1-ol und cis-4-Isopropyl-1-methyl-cyclohex-2-en-1-ol in einem Verhältnis von 1: 3,5 enthalten. Bei der Destillation an einer 30-cm-Füllkörperkolonne werden bei einem Siedepunkt von 85 - 102 °C, bei 14 mbar, 26 g einer Fraktion erhalten, die nach GC/GCMS 19 % trans-4-Isopropyl-1-methyl-cyclohex-2-en-1-ol und 67 % cis-4-Isopropyl-1-methyl-cyclohex-2-en-1-ol enthält.

Das Destillat (26 g) wird, in einem 0,1 Liter-Doppelwandgefäß mit Magnetrührer, Thermostatanschluss, Kühler und Stickstoffatmosphäre, zusammen mit 0,1 g 85-%iger Phosphorsäure vorgelegt. Dann werden 22 g (0,22 mol) Butylvinylether in 1 bis 2 Stunden bei 35 °C zugetropft. Zur Nachreaktion wird noch 2 Stunden bei 35 °C nachgerührt. Der Ansatz wird bei Raumtemperatur mit 5-%iger Sodalösung und dann mit 5-%iger Natriumchloridlösung gewaschen und anschließend bei 60 °C und 700 bis 5 mbar am Rotationsverdampfer eingedampft. Die Ausbeute beträgt 31,3 g.

30 g des Rohproduktes werden in einem 250-ml-Dreihalskolben mit Magnetrührer, 20 cm-Vigreuxkolonne und Destillationsbrücke zusammen mit 30 g Dibenzylether, und 1,5 g Capronsäure vorgelegt und bei 90 mbar aufgeheizt. Bei 160 - 175 °C werden die entstehenden Leichtsieder innerhalb von 3 Stunden bis zu einer Kopftemperatur von ca. 80 °C abdestilliert. Anschließend erfolgt die Destillation des Rohprodukts (Sumpftemperatur: 125 - 170 °C, Kopftemperatur: 65 - 90 °C, Vakuum: 1 mbar). Es werden 10 g abdestilliert.

Nach DB-1-GC bzw. GCMS sind 60 % Produkt (cis/trans-Isomere) entstanden. m/z : 43 (17), 55 (23), 67 (36), 81 (100), 93 (77), 110 (19), 119 (18), 136 (47), 165 (13), 180 (14)
Geruchsbeschreibung: Aldehydisch, grün, blumig, nach Maiglöckchen
m/z: 43 (19), 55 (27), 67 (40), 81 (100), 93 (95), 110 (24), 119 (21), 136 (51), 165 (15), 180 (13)
Geruchsbeschreibung: Grün, aldehydisch, blumig, nach Maiglöckchen.

### Ausführungsbeispiel 3: Parfümöl A

### Zusammensetzung des Parfümöls A1 bzw. A2:

| **Riechstoffe** | **Gewichtsteile** | |
|---|---|---|
| | **Parfümöl A1** (Vergleich) | **Parfümöl A2** (erfindungsgemäß) |
| ETHYL ACETOACETATE | 4,0 | 4,0 |
| HEXENOL CIS-3 10% DPG | 3,0 | 3,0 |
| HEXENYL ACETATE CIS-3 10% DPG | 3,0 | 3,0 |
| HEXENYL BENZOATE CIS-3 10% DPG | 3,0 | 3,0 |
| VERTOCITRAL 10% DPG | 5,0 | 5,0 |
| CYCLOGALBANAT^{®} 10% DPG | 3,0 | 3,0 |
| STYRALYL ACETATE 10% DPG | 6,0 | 6,0 |
| BERGAMOT OIL BERGAPTEN FREE FF | 6,0 | 6,0 |
| MANDARIN OIL DIST. DECOL. | 15,0 | 15,0 |
| METHYL ANTHRANILATE 10% DPG | 5,0 | 5,0 |
| RED BERRY EXTR. | 2,0 | 2,0 |
| DECALACTONE GAMMA | 2,0 | 2,0 |
| ALLYL CAPROATE 10% DPG | 5,0 | 5,0 |
| PRUNELLA TYPE BASE | 6,0 | 6,0 |
| HELIONAL | 20,0 | 20,0 |
| MUGETANOL | 10,0 | 10,0 |
| ETHYL LINALOOL | 35,0 | 35,0 |
| CITRONELLOL 950 | 10,0 | 10,0 |
| GERANIOL SUPER | 3,0 | 3,0 |
| ROSAPHEN^{®} | 8,0 | 8,0 |

| | | |
|---|---|---|
| CITRONELLYL ACETATE EXTRA | 2,0 | 2,0 |
| DAMASCONE BETA 10% DPG | 6,0 | 6,0 |
| BENZYL ACETATE | 10,0 | 10,0 |
| HEDION HC/30 | 30,0 | 30,0 |
| HEDIONE | 140,0 | 140,0 |
| HEXYL CINNAMIC ALDEHYDE ALPHA | 30,0 | 30,0 |
| LACTOJASMONE | 3,0 | 3,0 |
| BENZYL SALICYLATE | 80,0 | 80,0 |
| HEXENYL SALICYLATE CIS-3 | 30,0 | 30,0 |
| METHYL IONONE GAMMA COEUR | 3,0 | 3,0 |
| CLOVE BUD OIL | 1,0 | 1,0 |
| VANILLIN | 1,5 | 1,5 |
| COUMARIN | 1,0 | 1,0 |
| AMBERWOOD^{®} F | 5,0 | 5,0 |
| CASHMERAN | 8,0 | 8,0 |
| CEDRENE | 15,0 | 15,0 |
| ISO E SUPER NON DISCOLORING | 50,0 | 50,0 |
| BRAHMANOL^{®} | 25,0 | 25,0 |
| SANDALORE | 5,0 | 5,0 |
| AMBROXIDE | 2,0 | 2,0 |
| AMBRETTOLIDE | 5,0 | 5,0 |
| AURELIONE^{®} | 16,0 | 16,0 |
| GLOBALIDE^{®} | 24,0 | 24,0 |
| MACROLlDE^{®} SUPRA | 16,0 | 16,0 |
| INDOLE FF 10% DPG | 5,0 | 5,0 |
| 2-(5-Isopropyl-2-methyl-cyclohex-2-en-1-yl)acetaldehyd | | 50,0 |
| DIPROPYLENE GLYCOL | 332,5 | 282,5 |
| | 1.000,0 | 1.000,0 |

Bei einer Dosierung von 6 Gew.-% des Parfümöls A1 bzw. A2 ergibt sich folgender Befund: Im Vergleich zum Parfümöl A1 verstärkt der Zusatz von 2-(5-Isopropyl-2-methyl-cyclohex-2-en-1-yl)acetaldehyd im Parfümöl A2 blumige Aspekte.

### Ausführungsbeispiel 4: Parfümöl B

### Zusammensetzung des Parfümöls B1 bzw. B2:

| **Riechstoffe** | **Gewichtsteile** | |
|---|---|---|
| | **Parfümöl B1** (Vergleich) | **Parfümöl B2** (erfindungsgemäß) |
| NONADIENAL TRANS,CIS-2,6 5% TEC 20% DPG | 2,0 | 2,0 |
| ETHYL ACETOACETATE | 3,0 | 3,0 |
| FARENAL^{®} 10% DPG | 5,0 | 5,0 |
| VERTOCITRAL | 3,0 | 3,0 |
| CYCLOGALBANAT^{®} 10% DPG | 2,0 | 2,0 |
| STYRALYL ACETATE | 3,0 | 3,0 |
| MELONAL^{®} | 0,5 | 0,5 |
| DIHYDRO MYRCENOL | 15,0 | 15,0 |
| LINALYL ACETATE | 20,0 | 20,0 |
| LEMON OIL TERPENES FLAVOR WONF | 8,0 | 8,0 |
| EUCALYPTOL NAT. 10% DPG | 0,5 | 0,5 |
| HEXYL ACETATE | 1,5 | 1,5 |
| ISOAMYL ACETATE 10% DPG | 4,0 | 4,0 |
| PRENYL ACETATE 10% DPG | 4,0 | 4,0 |
| ALDEHYDE C14 SO-CALLED | 2,0 | 2,0 |
| ETHYL METHYL BUTYRATE-2 | 1,0 | 1,0 |
| ALLYL CYCLOHEXYL PROPIONATE | 2,0 | 2,0 |
| ALDEHYDE C16 SO-CALLED | 1,0 | 1,0 |
| FRAGOLANE^{®} | 0,5 | 0,5 |
| MAJANTOL^{®} | 25,0 | 25,0 |
| LINALOOL | 40,0 | 40,0 |
| DIMETHYL BENZYL CARBINOL | 10,0 | 10,0 |
| TERPINEOL PURE | 10,0 | 10,0 |
| PHENIRAT^{®} | 30,0 | 30,0 |
| CITRONELLOL 950 | 15,0 | 15,0 |
| GERANIOL 60 | 10,0 | 10,0 |
| CITRONELLYL ACETATE EXTRA | 2,0 | 2,0 |
| HEDIONE | 90,0 | 90,0 |
| HEXYL CINNAMIC ALDEHYDE ALPHA | 35,0 | 35,0 |
| HEXYL SALICYLATE | 160,0 | 160,0 |
| METHYL OCTIN CARBONATE 10% DPG | 2,0 | 2,0 |
| CALONE 1951 10% DPG | 1,0 | 1,0 |
| GALAXOLIDE 50% IN IPM | 20,0 | 20,0 |
| ANETHOL SUPRA 21,5 CELSIUS | 2,0 | 2,0 |
| AGRUMEX HC | 15,0 | 15,0 |
| ORYCLON SPECIAL | 40,0 | 40,0 |
| 2-(6-lsopropyl-3-methyl-cyclohex-2-en-1-yl)acetaldehyd | | 15,0 |
| DIPROPYLENE GLYCOL | 415,0 | 400,0 |
| | 1.000,0 | 1.000,0 |

Bei einer Dosierung von 0,5 Gew.-% des Parfümöls B1 bzw. B2 ergibt sich folgender Befund: Aufgrund des Anteils von 2-(6-lsopropyl-3-methyl-cyclohex-2-en-1-yl)acetaldehyd weist das Parfümöl B2 eine stärkere blumige Note auf als das Parfümöl B1 ohne 2-(6-Isopropyl-3-methyl-cyclohex-2-en-1-yl)acetaldehyd. Weiterhin zeigt Parfümöl B2 eine grünere Topnote als Parfümöl B1.

## Patentansprüche

1. Verbindungen der Formel (**I**) 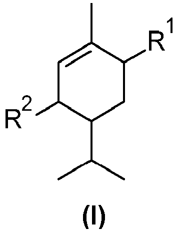 worin
R¹ für H oder -CH₂-CHO steht, und
R² für H oder -CH₂-CHO steht,
mit der Maßgabe, dass R¹ ≠ R² ist;
sowie deren Stereoisomere oder Mischungen daraus.

2. Verbindungen nach Anspruch 1, worin die Verbindungen der Formel (**I**) durch die nachfolgende Formel (**II**) oder Formel (**III**) wiedergegeben werden: 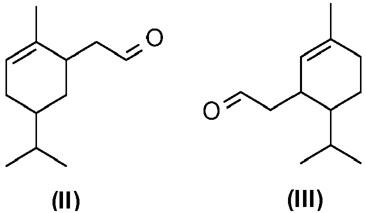 sowie deren Stereoisomere, insbesondere Diastereomere oder Enantiomere, oder Mischungen daraus.

3. Verbindungen nach Anspruch 2, worin die Verbindungen der Formel (**II**) durch die nachfolgende Formel (**IIa**) oder Formel (**IIb**) wiedergegeben werden: 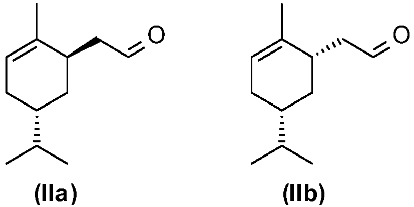 oder Mischungen daraus.

4. Verbindungen nach Anspruch 2, worin die Verbindungen der Formel (III) durch die nachfolgende Formel (**IIIa**) oder Formel (**IIIb**) wiedergegeben werden: 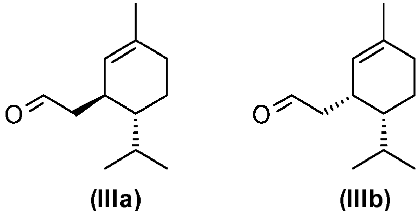 oder Mischungen daraus.

5. Mischung, umfassend oder bestehend aus (a) mindestens eine(r) Verbindung der Formel (**II**) oder ein Stereoisomer davon, und (b) mindestens eine(r) Verbindung der Formel (**III**) oder ein Stereoisomer davon nach einem der Ansprüche 2 bis 4.

6. Verfahren zur Herstellung einer Verbindung der Formel (**I**) oder der Formel (**II**) nach Anspruch 1, 2 oder 3, umfassend die folgenden Schritte:
(i) saure Umsetzung der Verbindung 2-Methyl-5-(1-methylethyl-)2-cyclohexen-1-ol (**IV**) mit wenigstens einem Vinylether (R-O) unter Erhalt eines Acetals (**V**);
(ii) Spaltung des in Schritt (i) erhaltenen Acetals (**V**) durch saure Katalyse in einen Vinylether (**VI**); und
(iii) thermische Überführung des in Schritt (ii) erhaltenen Vinylethers (**VI**) in den Aldehyd (**II**);
gemäß dem nachfolgenden Reaktionsschema:

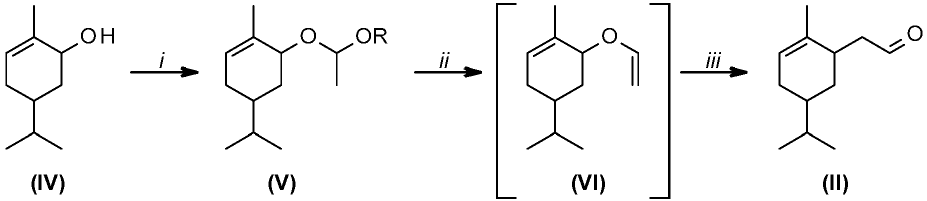

7. Verfahren zur Herstellung einer Verbindung der Formel (**I**) oder der Formel (**II**) nach Anspruch 1, 2 oder 3, umfassend die folgenden Schritte:
(i) saure Umsetzung der Verbindung 2-Methyl-5-(1-methylethenyl-)2-cyclohexen-1-ol (**VII**) mit wenigstens einem Vinylether (R-O) unter Erhalt eines Acetals (**VIII**);
(ii) Spaltung des in Schritt (iv) erhaltenen Acetals (**VIII**) durch saure Katalyse in einen Vinylether (**IX**);
(iii) thermische Überführung des in Schritt (v) erhaltenen Vinylethers (**IX**) in den Aldehyd (**X**); und
(iv) Hydrierung der terminalen Doppelbindung unter Erhalt des Aldehyds (**II**);
gemäß dem nachfolgenden Reaktionsschema:

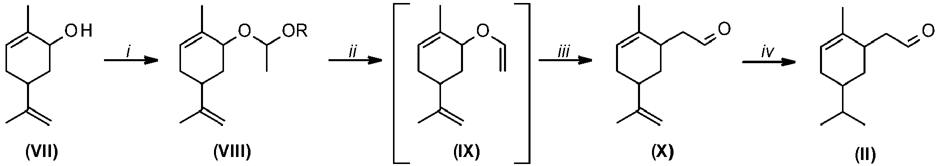

8. Verfahren zur Herstellung einer Verbindung der Formel (**I**) oder der Formel (**III**) nach Anspruch 1, 2 oder 4, umfassend die folgenden Schritte:
(i) saure Umsetzung der Verbindung 1-Methyl-4-(1-methylethyl-)2-cyclohexen-1-ol (**XI**) mit wenigstens einem Vinylether (R-O) unter Erhalt eines Acetals (**XII**);
(ii) Spaltung des in Schritt (vii) erhaltenen Acetals (**XII**) durch saure Katalyse in einen Vinylether (**XIII**); und
(iii) thermische Überführung des in Schritt (viii) erhaltenen Vinylethers (**XIII**) in den Aldehyd (**III**);
gemäß dem nachfolgenden Reaktionsschema:

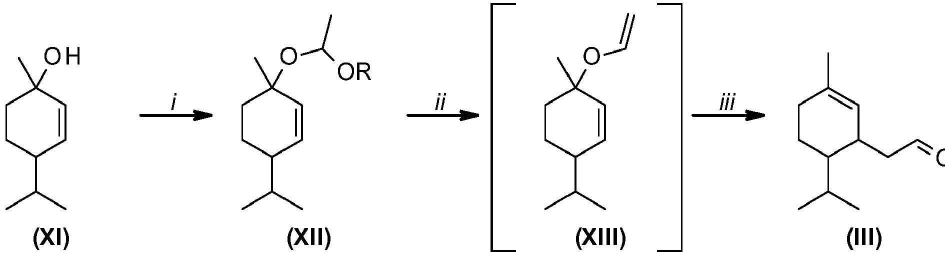

9. Verfahren zur Herstellung einer Verbindung der Formel (**I**) oder der Formel (**III**) nach Anspruch 1, 2 oder 4, umfassend die folgenden Schritte:
(i) saure Umsetzung der Verbindung 1-Methyl-4-(1-methylethenyl-)2-cyclohexen-1-ol (**XX**) mit wenigstens einem Vinylether (R-O) unter Erhalt eines Acetals (**XXI**);
(ii) Spaltung des in Schritt (vii) erhaltenen Acetals (**XXI**) durch saure Katalyse in einen Vinylether (**XXII**);
(iii) thermische Überführung des in Schritt (viii) erhaltenen Vinylethers (**XXII**) in den Aldehyd (**XXIII**); und
(iv) Hydrierung der terminalen Doppelbindung unter Erhalt des Aldehyds (**III**);
gemäß dem nachfolgenden Reaktionsschema:

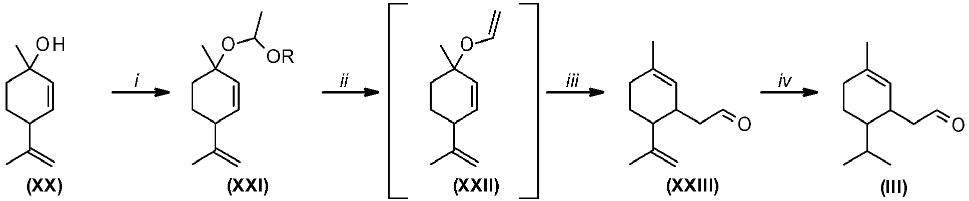

10. Verfahren zur Herstellung einer Verbindung der Formel (**II**) oder einer Verbindung der Formel (**III**) nach einem der Ansprüche 6 bis 9, worin der Rest R des Vinylethers (R-O) in Schritt (i) ausgewählt ist aus der Gruppe, die besteht aus linearem C1- bis C20-Alkylrest, verzweigtem C1- bis C20-Alkylrest, Cyclopentylrest und Cyclohexylrest.

11. Verwendung mindestens einer Verbindung der Formeln (**I**), (**II**) oder (**III**) oder ein Stereoisomer davon oder einer Mischung aus den Verbindungen der Formeln (**I**), (**II**) oder (**III**) oder dessen Stereoisomere nach einem der vorangehenden Ansprüche als Riechstoff oder zur Herstellung einer Riechstoffzubereitung.

12. Riechstoff oder Riechstoffzubereitung, umfassend wenigstens eine Verbindung der Formeln (**I**), (**II**) oder (**III**) oder ein Stereoisomer davon nach einem der vorangehenden Ansprüche, vorzugsweise in einer sensorisch wirksamen Menge.

13. Riechstoffzubereitung nach dem vorhergehenden Anspruch, weiterhin umfassend einen oder mehrere zusätzliche Riechstoff(e) und/oder Aromastoff(e), wobei der oder die mehreren zusätzliche(n) Riechstoff(e) und/oder Aromastoff(e) ausgewählt ist/sind aus der Gruppe, die besteht aus: Extrakten aus natürlichen Rohstoffen, vorzugsweise Etherische Öle, Concretes, Absolues, Resine, Resinoide, Balsame, Tinkturen und/oder Einzel-Riechstoffen, vorzugsweise aus der Gruppe der Kohlenwasserstoffe, der aliphatischen Alkohole, der aliphatischen Aldehyde und deren Acetale, der aliphatischen Ketone und deren Oxime, der aliphatischen schwefelhaltigen Verbindungen, der aliphatischen Nitrile, der Ester von aliphatischen Carbonsäuren, der acyclischen Terpenalkohole, der acyclischen Terpenaldehyde und -ketone, der cyclischen Terpenalkohole, der cyclischen Terpenaldehyde und -ketone, der cyclischen Alkohole, der cycloaliphatischen Alkohole, der cyclischen und cycloaliphatischen Ether, der cyclischen und makrocyclischen Ketone, der cycloaliphatischen Aldehyde, der cycloaliphatischen Ketone, der Ester cyclischer Alkohole, der Ester cycloaliphatischer Alkohole, der aliphatischen Alkohole, der Ester von aliphatischen Alkoholen und aliphatischen Carbonsäuren, der aliphatischen Ether, der aromatischen und aliphatischen Aldehyde, der aromatischen und aliphatischen Ketone, der aromatischen und aliphatischen Carbonsäuren und deren Ester, der stickstoffhaltigen aromatischen Verbindungen, der Phenole, Phenylether und Phenylester, der heterocyclischen Verbindungen, und der Lactone.

14. Riechstoffzubereitung nach Anspruch 12 oder Anspruch 13, wobei die Gesamtmenge der Verbindungen der Formeln (**I**), (**II**) oder (**III**) nach einem der vorangehenden Ansprüche 0,00001 bis 5 Gew.-%, vorzugsweise 0,00001 bis 1 Gew.-%, und besonders bevorzugt 0,0005 bis 0,01 Gew.-%, beträgt, bezogen auf das Gesamtgewicht der Riechstoffzubereitung.

15. Parfümiertes Produkt, umfassend einen Riechstoff oder eine Riechstoffzubereitung nach einem der Ansprüche 12 bis 14, und gegebenenfalls ein Ergänzungsmittel, bevorzugt einen Trägerstoff.

16. Parfümiertes Produkt nach dem vorangehenden Anspruch, umfassend darüber hinaus einen oder mehrere Zusatzstoffe, ausgewählt aus der Gruppe, die besteht aus: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

17. Parfümiertes Produkt nach Anspruch 15 oder Anspruch 16, ausgewählt aus der Gruppe, die besteht aus: Wasch- und Reinigungsmitteln, Hygiene- oder Pflegeprodukten, insbesondere aus dem Bereich der Körper- und Haarpflege, der Kosmetik, des Haushalts, Fine Fragrance (Parfüm), Air Care und Lufterfrischer.

## Claims

1. Compounds of formula (I) 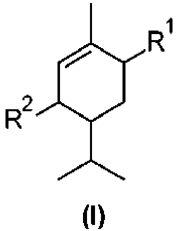 wherein
R¹ is H or -CH₂-CHO, and
R² is H or -CH₂-CHO,
provided that R¹ ≠ R²;
and stereoisomers or mixtures thereof.

2. Compounds according to claim 1, wherein the compounds of formula (I) are represented by the following formula (II) or formula (III): 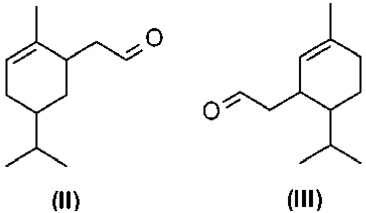 and their stereoisomers, in particular diastereomers or enantiomers, or mixtures thereof.

3. Compounds according to claim 2, wherein the compounds of formula (II) are represented by the following formula (IIa) or formula (IIb): 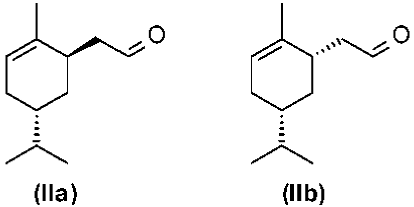 or mixtures thereof.

4. Compounds according to claim 2, wherein the compounds of formula (III) are represented by the following formula (IIIa) or formula (IIIb): 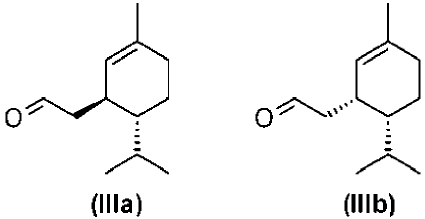 or mixtures thereof.

5. Mixture comprising or consisting of (a) at least one compound of formula (II) or a stereoisomer thereof, and (b) at least one compound of formula (III) or a stereoisomer thereof according to any one of claims 2 to 4.

6. Method for producing of a compound of formula (I) or of formula (II) according to claim 1, 2 or 3, comprising the following steps:
(i) acidic reaction of the compound 2-methyl-5-(1-methylethyl-)2- cyclohexen-1-ol (IV) with at least one vinyl ether (R-O) to give an acetal (V);
(ii) cleavage of the acetal (V) obtained in step (i) by acid catalysis into a vinyl ether (VI); and
(iii) thermal conversion of the vinyl ether (VI) obtained in step (ii) into the aldehyde (II);
according to the following reaction scheme:

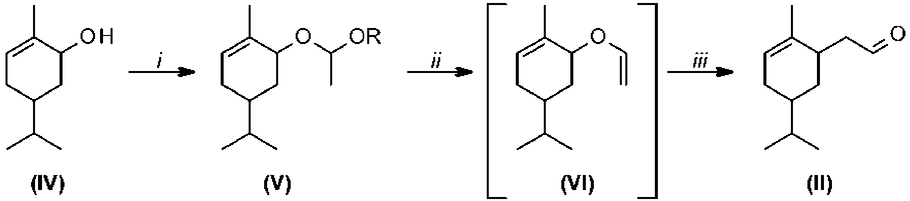

7. Method for producing of a compound of formula (I) or of formula (II) according to claim 1, 2 or 3, comprising the following steps:
(i) acidic reaction of the compound 2-methyl-5-(1-methylethenyl-)2- cyclohexen-1-ol (VII) with at least one vinyl ether (R-O) to give an acetal (VIII);
(ii) cleavage of the acetal (VIII) obtained in step (iv) by acid catalysis into a vinyl ether (IX);
(iii) thermal conversion of the vinyl ether (IX) obtained in step (v) into the aldehyde (X); and
(iv) hydrogenation of the terminal double bond to obtain the aldehyde (II);
according to the following reaction scheme:

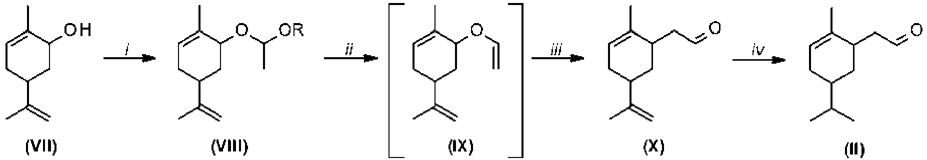

8. Method for producing of a compound of formula (I) or of formula (III) according to claim 1, 2 or 4, comprising the following steps:
(i) acidic reaction of the compound 1-methyl-4-(1-methylethyl-)2- cyclohexen-1-ol (XI) with at least one vinyl ether (R-O) to give an acetal (XII);
(ii) cleaving the acetal (XII) obtained in step (vii) by acid catalysis into a vinyl ether (XIII); and
(iii) thermal conversion of the vinyl ether (XIII) obtained in step (viii) into the aldehyde (III);
according to the following reaction scheme:

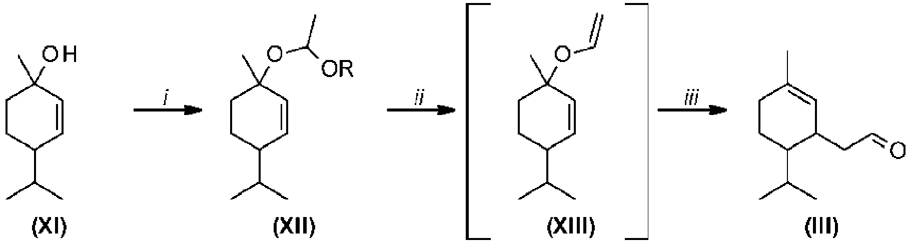

9. Method for producing of a compound of formula (I) or of formula (III) according to claim 1, 2 or 4, comprising the following steps:
(i) acidic reaction of the compound 1-methyl-4-(1-methylethenyl-)2- cyclohexen-1-ol (XX) with at least one vinyl ether (R-O) to give an acetal (XXI);
(ii) cleavage of the acetal (XXI) obtained in step (vii) by acid catalysis into a vinyl ether (XXII);
(iii) thermal conversion of the vinyl ether (XXII) obtained in step (viii) into the aldehyde (XXIII); and
(iv) hydrogenation of the terminal double bond to obtain the aldehyde (III);
according to the following reaction scheme:

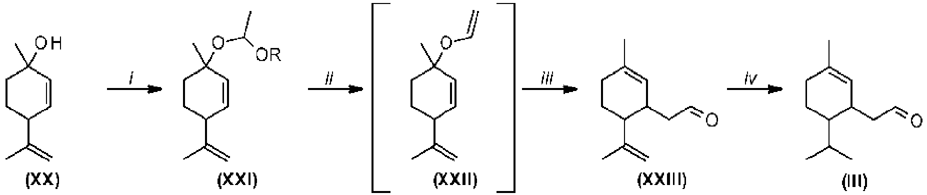

10. Method for producing of a compound of formula (II) or a compound of formula (III) according to any one of claims 6 to 9, wherein the radical R of the vinyl ether (R-O) in step (i) is selected from the group consisting of linear C1 to C20 alkyl radical, branched C1 to C20 alkyl radical, cyclopentyl radical and cyclohexyl radical.

11. Use of at least one compound of formulae (I), (II) or (III) or a stereoisomer thereof or a mixture of the compounds of formulae (I), (II) or (III) or stereoisomers thereof according to any one of the preceding claims as a perfuming ingredient or for the preparation of a perfuming composition.

12. Perfume or perfume preparation comprising at least one compound of formula (I), (II) or (III) or a stereoisomer thereof according to any one of the preceding claims, preferably in a sensory effective amount.

13. Perfume preparation according to the preceding claim, further comprising one or more additional perfume(s) and/or aroma(s), wherein the one or more additional perfume(s) and/or aroma(s) is/are selected from the group consisting of: extracts of natural raw materials, preferably essential oils, concretes, absolues, resines, resinoids, balsams, tinctures and/or single perfumes, preferably from the group of hydrocarbons, aliphatic alcohols, aliphatic aldehydes and their acetals, aliphatic ketones and their oximes, aliphatic sulphur-containing compounds, aliphatic nitriles, esters of aliphatic carboxylic acids, acyclic terpene alcohols, acyclic terpene aldehydes and ketones, cyclic terpene alcohols, cyclic terpene aldehydes and ketones, cyclic alcohols, cycloaliphatic alcohols, of cyclic and cycloaliphatic ethers, cyclic and macrocyclic ketones, cycloaliphatic aldehydes, cycloaliphatic ketones, esters of cyclic alcohols, esters of cycloaliphatic alcohols, aliphatic alcohols, esters of aliphatic alcohols and aliphatic carboxylic acids, aliphatic ethers, aromatic and aliphatic aldehydes, aromatic and aliphatic ketones, aromatic and aliphatic carboxylic acids and their esters, nitrogen-containing aromatic compounds, phenols, phenyl ethers and phenyl esters, heterocyclic compounds, and lactones.

14. Perfume preparation according to claim 12 or claim 13, wherein the total amount of the compounds of formulae (I), (II) or (III) according to any one of the preceding claims is 0.00001 to 5% by weight, preferably 0.00001 to 1% by weight, and more preferably 0.0005 to 0.01% by weight, based on the total weight of the perfume preparation.

15. Perfumed product comprising a perfuming ingredient or a perfuming preparation according to any one of claims 12 to 14, and optionally a supplement, preferably a carrier.

16. Perfumed product according to the preceding claim, further comprising one or more additives selected from the group consisting of: Preservatives, Abrasives, Anti-acne agents, Anti-ageing agents, Antibacterial agents, Anticellulitis agents, Anti-dandruff agents, Anti-inflammatory agents, Anti-irritant agents, Anti-irritant agents, Antimicrobial agents, antioxidants, astringents, antiperspirants, antiseptics, antistatics, binders, buffers, carriers, chelating agents, cell stimulants, cleansing agents, conditioning agents, depilatories, surfactants, deodorants, antiperspirants, emollients, emulsifiers, enzymes, essential oils, fibres, film formers, fixatives, foaming agents, foam stabilisers, anti-foaming agents, foam boosters, fungicides, gelling agents, gel-forming agents, hair care agents, hair shaping agents, hair smoothing agents, moisturising agents, moisturising substances, bleaching agents, strengthening agents, stain removing agents, optical brightening agents, impregnating agents, soil-repellent agents, friction-reducing agents, lubricants, moisturisers, ointments, opacifiers, plasticising agents, covering agents, polishes, glossing agents, polymers, powders, proteins, refatting agents, abrasives, silicones, skin soothing agents, skin cleansing agents, skin conditioning agents, skin healing agents, skin whitening agents, skin protecting agents, skin softening agents, cooling agents, skin cooling agents, warming agents, skin warming agents, stabilisers, UV absorbing agents, UV filters, detergents, fabric softeners, suspending agents, skin tanning agents, thickening agents, vitamins, oils, waxes, fats, phospholipids, saturated fatty acids, mono- or polyunsaturated fatty acids, a-hydroxy acids, polyhydroxy fatty acids, liquefiers, dyes, colour-protecting agents, pigments, anti-corrosives, flavourings, aromatic substances, polyoiesis, surfactants, electrolytes, organic solvents or silicone derivatives.

17. Perfumed product according to claim 15 or claim 16, selected from the group consisting of: Detergents, hygiene or care products, in particular in the field of body and hair care, cosmetics, household, fine fragrance (perfume), air care and air freshener.

## Revendications

1. Composés de formule (I) 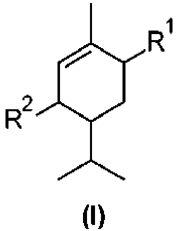 où
R¹ représente H ou -CH2-CHO, et
R² représente H ou -CH2-CHO,
à condition que R¹ ≠ R2 ;
ainsi que leurs stéréoisomères ou des mélanges de ceux-ci.

2. Composés selon la revendication 1, où les composés de formule (I) sont représentés par la formule (II) ou la formule (III) suivantes : 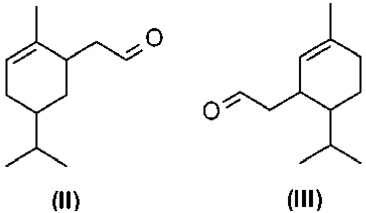 ainsi que leurs stéréoisomères, en particulier les diastéréomères ou énantiomères, ou des mélanges de ceux-ci.

3. Composés selon la revendication 2, où les composés de formule (II) sont représentés par la formule (IIa) ou la 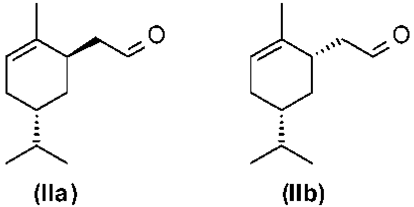 ou des mélanges de ceux-ci.

4. Composés selon la revendication 2, où les composés de formule (III) sont représentés par la formule (IIIa) ou la formule (IIIb) suivantes : 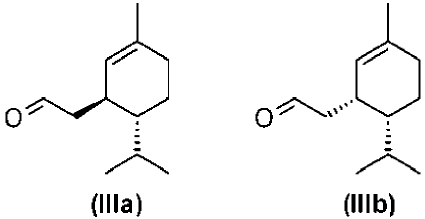 ou des mélanges de ceux-ci.

5. Mélange, comprenant ou consistant en (a) au moins un composé de formule (II) ou un stéréoisomère de celui-ci, et (b) au moins un composé de formule (III) ou un stéréoisomère de celui-ci selon l'une des revendications 2 à 4.

6. Procédé de préparation d'un composé de formule (I) ou de formule (II) selon la revendication 1, la revendication 2 ou la revendication 3, comprenant les étapes suivantes :
(i) réaction acide du composé 2-méthyl-5-(1-méthyléthyl-)2-cyclohexène-1-ol (IV) avec au moins un éther vinylique (R-O) pour obtenir un acétal (V) ;
(ii) décomposition de l'acétal (V) obtenu lors de l'étape (i) en un éther vinylique (VI) par catalyse acide ; et
(iii) conversion thermique en l'aldéhyde (II) de l'éther vinylique (VI) obtenu lors de l'étape (ii) ;
conformément au schéma de réaction ci-dessous :

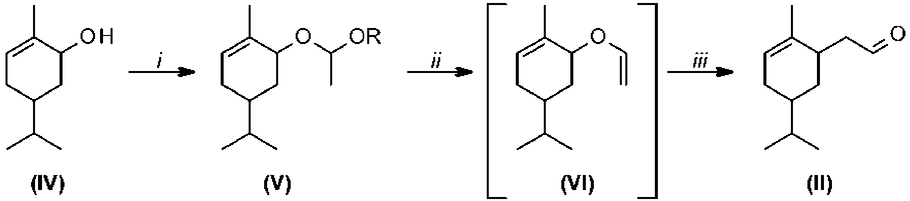

7. Procédé de préparation d'un composé de formule (I) ou de formule (II) selon la revendication 1, la revendication 2 ou la revendication 3, comprenant les étapes suivantes :
(i) réaction acide du composé 2-méthyl-5-(1-méthyléthényl-)2-cyclohexène-1-ol (VII) avec au moins un éther vinylique (R-O) pour obtenir un acétal (VIII) ;
(ii) décomposition de l'acétal (VIII) obtenu lors de l'étape (iv) en un éther vinylique (IX) par catalyse acide ;
(iii) conversion thermique en l'aldéhyde (X) de l'éther vinylique (IX) obtenu lors de l'étape (v); et
(iv) hydrogénation de la double liaison terminale, avec obtention de l'aldéhyde (II) ;
conformément au schéma de réaction ci-dessous :

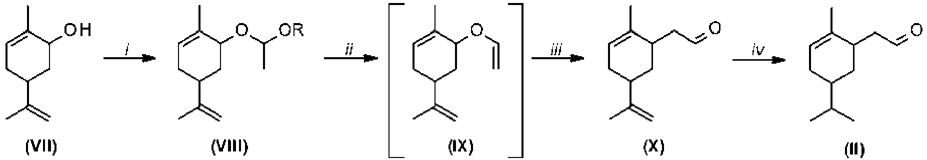

8. Procédé de préparation d'un composé de formule (I) ou de formule (III) selon la revendication 1, la revendication 2 ou la revendication 4, comprenant les étapes suivantes :
(i) réaction acide du composé 1-méthyl-4-(1-méthyléthyl-)2-cyclohexène-1-ol (XI) avec au moins un éther vinylique (R-O) pour obtenir un acétal (XII) ;
(ii) décomposition de l'acétal (XII) obtenu lors de l'étape (vii) en un éther vinylique (XIII) par catalyse acide ; et
(iii) conversion thermique en l'aldéhyde (III) de l'éther vinylique (XIII) obtenu lors de l'étape (viii);
conformément au schéma de réaction ci-dessous :

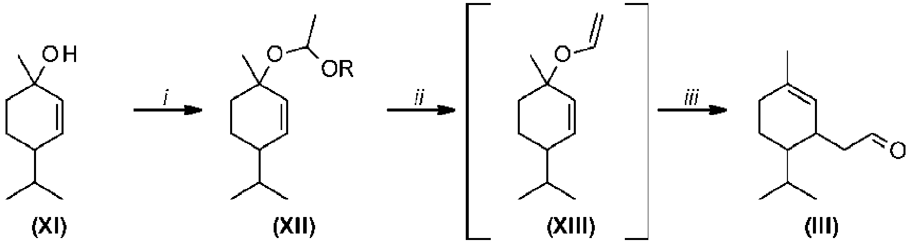

9. Procédé de préparation d'un composé de formule (I) ou de formule (III) selon la revendication 1, la revendication 2 ou la revendication 4, comprenant les étapes suivantes :
(i) réaction acide du composé 1-méthyl-4-(1-méthyléthényl-)2-cyclohexène-1-ol (XX) avec au moins un éther vinylique (R-0) pour obtenir un acétal (XXI) ;
(ii) décomposition de l'acétal (XXI) obtenu lors de l'étape (vii) en un éther vinylique (XXII) par catalyse acide ;
(iii) conversion thermique en l'aldéhyde (XXIII) de l'éther vinylique (XXII) obtenu lors de l'étape (viii); et
(iv) hydrogénation de la double liaison terminale, avec obtention de l'aldéhyde (III) ;
conformément au schéma de réaction ci-dessous :

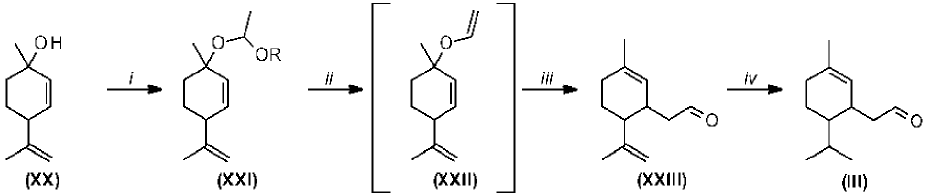

10. Procédé de préparation d'un composé de formule (II) ou d'un composé de formule (III) selon l'une des revendications 6 à 9, où le radical R de l'éther vinylique (R-O) lors de l'étape (i) est sélectionné dans le groupe constitué par un radical alkyle linéaire en C1 à C20, un radical alkyle ramifié en C1 à C20, un radical cyclopentyle et un radical cyclohexyle.

11. Utilisation d'au moins un composé de formule (I), (II) ou (III) ou d'un stéréoisomère de celui-ci ou d'un mélange des composés de formule (I), (II) ou (III) ou de leurs stéréoisomères selon l'une des revendications précédentes, en tant que substance odorante ou pour la préparation d'une composition de parfum.

12. Substance odorante ou composition de parfum comprenant au moins un composé de formule (I), (II) ou (III) ou un stéréoisomère de celui-ci selon l'une des revendications précédentes, de préférence en une teneur efficace du point de vue sensoriel.

13. Composition de parfum selon la revendication précédente, comprenant en outre une ou plusieurs substances odorantes et/ou substances aromatiques additionnelles, où la ou les substances odorantes et/ou substances aromatiques additionnelles sont sélectionnées dans le groupe comprenant : extraits de matières premières naturelles, de préférence huiles essentielles, concrétions, absolues, résines, de résinoïdes, baumes, teintures et/ou substances odorantes individuelles, de préférence dans le groupe des hydrocarbures, alcools aliphatiques, aldéhydes aliphatiques et leurs acétals, cétones aliphatiques et leurs oximes, composés aliphatiques contenant du soufre, nitriles aliphatiques, esters d'acides carboxyliques aliphatiques, alcools terpéniques acycliques, aldéhydes et cétones terpéniques acycliques, alcools terpéniques cycliques, aldéhydes et cétones terpéniques cycliques, alcools cycloaliphatiques, éthers cycliques et cycloaliphatiques, cétones cycliques et macrocycliques, aldéhydes cycloaliphatiques, cétones cycloaliphatiques, esters d'alcools cycliques, esters d'alcools cycloaliphatiques, alcools aliphatiques, esters d'alcools aliphatiques et d'acides carboxyliques aliphatiques, éthers aliphatiques, aldéhydes aromatiques et aliphatiques, cétones aromatiques et aliphatiques, acides carboxyliques aromatiques et aliphatiques et leurs esters, composés aromatiques azotés, phénols, éthers phényliques et esters phényliques, composés hétérocycliques, et lactones.

14. Composition de parfum selon la revendication 12 ou la revendication 13, où la teneur totale des composés de formule (I), (II) ou (III) selon l'une des revendications précédentes est comprise entre 0,00001 et 5 % en poids, avantageusement entre 0,00001 et 1 % en poids, et de préférence entre 0,0005 et 0,01 % en poids, par rapport au poids total de la composition de parfum.

15. Produit parfumé comprenant une substance odorante ou une composition de parfum selon l'une des revendications 12 à 14, et éventuellement un agent complémentaire, de préférence un excipient.

16. Produit parfumé selon la revendication précédente, comprenant en outre un ou plusieurs additifs sélectionnés dans le groupe comprenant : conservateurs, abrasifs, anti-acnéiques, agents antivieillissement cutané, antibactériens, agents anticellulite, antipelliculaires, anti-inflammatoires, anti-irritants, apaisants, antimicrobiens, antioxydants, astringents, antitranspirants, antiseptiques, antistatiques, liants, tampons, excipients, chélatants, stimulants cellulaires, agents nettoyants, agents de soin, dépilatoires, tensioactifs, déodorants, antiperspirants, adoucissants, émulsifiants, enzymes, huiles essentielles, fibres, agents filmogènes, fixateurs, agents moussants, stabilisateurs de mousse, agents antimousse, boosters de mousse, fongicides, agents gélifiants, agents formateurs de gel, agents de soins capillaires, agents de mise en forme des cheveux, agents de lissage des cheveux, agents hydratants, substances humectantes, substances hydratantes, agents décolorants, agents fortifiants, agents détachants, agents d'éclaircissement optique, agents d'imprégnation, agents antisalissants, agents réduisant le frottement, lubrifiants, crèmes hydratantes, pommades, opacifiants, agents plastifiants, agents couvrants, polish, agents brillants, polymères, poudres, protéines, agents relipidants, agents abrasifs, silicones, agents apaisants pour la peau, agents de nettoyage de la peau, agents de soin de la peau, agents de guérison de la peau, agents d'éclaircissement de la peau, agents de protection de la peau, agents adoucissants pour la peau, agents de refroidissement, agents de refroidissement de la peau, agents chauffants, agents de réchauffement de la peau, stabilisateurs, agents absorbant les UV, filtres UV, détergents, adoucissants, agents de suspension, agents de bronzage cutané, épaississants, vitamines, huiles, cires, graisses, phospholipides, acides gras saturés, acides gras mono- ou polyinsaturés, acides a-hydroxy, acides gras polyhydroxy, fluidifiants, colorants, agents de protection des couleurs, pigments, anticorrosifs, arômes, saveurs, substances odorantes, polyols, tensioactifs, électrolytes, solvants organiques ou dérivés de silicone.

17. Produit parfumé selon la revendication 15 ou la revendication 16, sélectionné dans le groupe comprenant : produits lavants et nettoyants, produits d'hygiène ou de soins, en particulier dans le domaine des soins corporels et capillaires, des cosmétiques, de la maison, de la parfumerie fine, de l'assainissement de l'air et des désodorisants.
